# EUROPEAN PATENT SPECIFICATION

(11) **EP 0 282 965 B1**
(45) Date of publication and mention of the grant of the patent: **20.07.1994**
(21) Application number: 88104071.1
(22) Date of filing: 15.03.1988
(51) Int. Cl.: A61K 39/02

(54) **Treponema hyodysenteriae antigen and uses therefor**
Treponema hyodysenteriae-Antigen und seine Verwendungen
Antigène Treponema hyodysenteriae et ses utilisations

(30) Priority: 17.03.1987 US 26781
(43) Date of publication of application: 21.09.1988
(73) Proprietor: ML TECHNOLOGY VENTURES, L.P., New York New York 10080 (US)
(72) Inventor: Gabe, Jeffrey D., San Francisco, CA 94118 (US); Mc Caman, Michael, San Bruno, CA 94066 (US)
(74) Representative: Perry, Robert Edward

(56) References cited:
- EP-A- 0 009 577
- EP-A- 0 155 790
- MICROBIOLOGICA, vol. 10, 1987; G. DETTORI et al., pp. 1-8#
- ABSTRACTS ANNUAL MEETING AMERICAN SOCIETY MICROBIOLOGY, vol. 87, no. 0, 1987; R.D. HUBBARD et al., p. 117, no. E-85#
- CHEMICAL ABSTRACTS, vol. 106, no. 5, 02 February 1987, Columbus, OH (US); L.A. JOENS et al., p. 378, no. 31066n#
- CHEMICAL ABSTRACTS, vol. 110, no. 7, 13 February 1989, Columbus, OH (US); S.N. CHATFIELD et al., p. 364, no. 54187z#

## Description

This invention relates to Treponema hyodysenteriae (sometimes hereinafter "Th") antigens and their use.

Swine dysentery (sometimes hereinafter "SD") is a severe infectious disease found in all major pig-rearing countries. The symptoms of swine dysentery are severe mucohemoragic diarrhoea, dehydration and weight loss.

It has been discovered that SD is caused by Th, an anaerobic, B-hemolytic spirochete. SD generally results from ingestion of faeces containing Th from acutely-infected or asymptomatic carrier pigs, or by faeces spread by farm equipment or handlers.

There have been numerous attempts to provide a vaccine against Th, which have generally involved use of the whole organism.

Microbiologica 10 (January 1987) p.1-8, discloses cross-reactivity between human and swine intestinal treponemes. Protein bands are identified using antibodies raised against whole cells.

EP-A-0155790 and EP-A-0009577 each disclose a vaccine containing killed pathogenic Th cells.

Abstracts Annual Meeting American Society Microbiology 87 (1987; meeting: March 1-6) abstract no. E-85, p.117, discloses the extraction of outer membrane antigens from Th by the use of chaotropic agents. The antigens of interest for protection of pigs against SD is disclosed as having a molecular weight of 14 kDa.

Chemical Abstracts 106 (February 2, 1987):31066n, and Infection and Immunity 54 (1986) p.893-896, disclose the identification of proteins from sonicated Th cells. A 16 kDa antigen is disclosed as probably being responsible for the protection of pigs against SD.

According to the present invention, a vaccine for protection against SD comprises a Th antigen having a molecular weight of 19 to 90 kDa, or an active fragment thereof, the vaccine being essentially free of Th cells. Such an antigen, essentially free of Th cells, can be used for the manufacture of a medicament for protection against SD. If desired, a mixture of the antigens may be used.

The molecular weight of the antigen is preferably at least 25 kDa, and preferably also no greater than 65 kDa. More preferably, the antigen is selected from those of 29, 30, 31, 34, 36, 38, 39, 42, 44 or 60 kDa, and mixtures thereof, e.g. from those of 30, 36 or 60 kDa, or from those of 29, 31, 34, 38, 39, 42 or 44 kDa. The characteristic molecular weights are obtained by discontinuous polyacrylamide gel electrophoresis using the SD buffer system described by Laemmli, Nature 227; 680-85 (London 1970) with an acrylamide concentration of 10 to 17% and a bis-acrylamide to acrylamide ratio of 1:29.

The antigens generally are also immunoreactive with serum obtained from swine which are convalescing from SD.

The antigen may be obtained by solubilising a Th organism, e.g. the surface or membrane antigens of the Th organism, with a detergent or chaotropic agent, without lysing or rupturing the organism. In accordance with a particularly preferred embodiment, the T. hyodysenteriae protein antigen is solubilized by treating the unlysed or whole organism, although, in some cases it may be possible to obtain such a soluble surface antigen from a lysed or sonicated organism.

The solubilization is preferably accomplished by the use of an appropriate detergent and/or a chaotropic agent which does not destroy the antigenic characteristics of the T. hyodysenteriae protein antigen. The detergent or surfactant may be any one of a wide variety of detergents including cationic, non-ionic and anionic surfactants. Such surfactants are generally known in the art, and include condensation products with ethylene oxide, various alkali metal salts of soaps, sulfates, or sulfonated oils, various amines, quaternary salts and the like. Preferred detergents include alkali dodecyl sulfate, laurolyl-sarcosine; non-ionic condesation products of ethylene oxide (for example Tween 20), etc..

As representative examples of suitable chaotropic agents, there may be mentioned: urea, lithium chloride, guanidine. HCl, and the like.

The solubilizing agent, whether it is a surfactant or chaotropic agent, is preferably employed in an amount which is sufficient to solubilize the antigen, without disrupting or lysing the whole organisms. In general, the solubilizing agent is employed in a solubilizing agent to organism weight ratio of from 0.05 to 1 to about 1.0 to 1, and preferably from about 0.1 to 1 to 0.5 to 1.

The solubilization is effected at a temperature which does not adversely affect the antigen, with such temperature generally being in the order from 15°C to 25°C. Similarly, the pH is selected to maintain stability, with the pH generally being no greater than 7.0, and most generally being from 4.5 to 5.0.

The treatment of the organism is for a time sufficient to effect solubilization of the antigen, and in general, such time is in the order of from 1 to 2 hours; however, in some cases longer or shorter times may be applicable.

The selection of optimum conditions for recovering the desired surface antigen by a solubilization technique is deemed to be within the scope of those skilled in the art from the teachings herein. As hereinabove indicated, the conditions and solubilizing agent are such that the antigens are obtained without lysing or rupturing the whole cell.

Thus, a characteristic of the antigen or antigens which are suitable for use in a vaccine in accordance with the present invention is that the T. hyodysenteriae antigen corresponds to an antigen or antigens which are solubilized by treating the T. hyodysenteriae organism with a detergent in a manner which does not lyse or rupture the organism, even though the antigen or antigens may be obtained by a procedure different than the described solubilization procedure.

It is to be understood, however, that even though a characteristic of the hereinabove described antigen is that it is separated from the whole organism as a soluble fraction when the whole organism is treated with a detergent under conditions which do not rupture or lyse the organism, the antigen or antigens after recovery thereof are not necessarily in soluble form when used in the vaccine or prior to formulating the vaccine. Similarly, although the T. hyodysenteriae antigen(s) employed in formulating the vaccine is preferably derived from the membrane of the organism by use of a solubilizing agent, as hereinabove described, it is to be understood that it is possible to obtain such antigen or antigens by other techniques or from other portions of the organism by the solubilization technique described.

In accordance with an embodiment of the present invention, the vaccine includes the 19Kda antigen or fragment thereof alone or in combination with other antigens (or fragments thereof), as hereinabove described. Although the 19Kda antigen is present in the soluble fraction recovered by treating the T. hyodysenteriae organism with a detergent and/or chaotropic agent, without lysing or rupturing the organism, the major portion of the 19Kda antigen is not solubilized and remains with the organism (in particular the membrane of the organism). The 19Kda antigen may be recovered from the organism by procedures known in the art; for example by disrupting the cells and the use of electrophoresis, prior or subsequent to extracting the soluble antigen fraction from the organism.

Similarly, it is possible to employ a fragment of one or more of the hereinabove described antigens in producing a vaccine of the present invention in place of or in conjunction with one or more of such antigens. The term fragment of the antigen as used herein is a fragment of the antigen which (1) includes an epitope which will produce an antibody which recognizes such antigen and (2) will immunoreact with serum of swine convalescing from swine dysentery. The fragment will have a molecular weight lower than the molecular weight of the antigen described.

Thus, in accordance with an aspect of the present invention, there is provided a protein or proteins which are essentially free of T. hyodysenteriae (T. hyo.) cells which protein or proteins produce an antibody or antibodies which recognizes at least one T. hyo. antigen having a molecular weight from about 19 kda to about 90 kda and preferably such T. hyo. antigen which is recognized has a molecular weight of at least 25 kda and most generally the molecular weight does not exceed 65 kda. In a particularly preferred embodiment such protein or proteins produce an antibody which recognizes one or more of the T. hyo. antigens having the following molecular weights: 19 kda; 29 kda; 30 kda; 31 kda; 34 kda; 36 kda; 38 kda; 39 kda; 42 kda; 44 kda; and 60 kda. As hereinabove indicated, in general, such protein or proteins will immunoreact with serum of swine convalescing from swine dysentery. Such protein may be the corresponding T. hyo. antigen and/or a fragment and/or derivative thereof. Such protein or proteins, as hereinafter indicated, may be used in combination with a physiologically acceptable vehicle as a vaccine for protecting swine against swine dysentery.

The T. hyodysenteriae antigen and/or fragment in conjunction with a physiologically acceptable carrier is employed as a vaccine to provide protection against swine dysentery and in particular swine dysentery induced by T. hyodysenteriae. The T. hyodysenteriae protein antigen(s) and/or fragment(s) thereof are employed in the vaccine in an amount effective to provide protection against swine dysentery. In general, each dose of the vaccine contains at least 5 micrograms and preferably at least 100 micrograms of such antigen(s) and/or fragments of the antigen. In most cases, the vaccine does not include such antigen(s) and/or fragments in an amount greater than 20 milligrams.

The term "protection" or "protecting" when used with respect to the vaccine for swine dysentery described herein means that the vaccine prevents swine dysentery and/or reduces the severity of swine dysentery.

If multiple doses are given, in general they would not exceed 3 doses over a six week period.

The carrier which is employed in conjunction with the T. hyodysenteriae protein antigen may be any one of a wide variety of carriers. As representative examples of suitable carriers, there may be mentioned: mineral oil, alum, synthetic polymers, etc. Carriers for vaccines are well known in the art and the selection of a suitable carrier is deemed to be within the scope of those skilled in the art from the teachings herein. The selection of a suitable carrier is also dependent upon the manner in which the vaccine is to be administered. The vaccine may be in the form of an injectable dose and may be administered intra-muscularly, intravenously, or by sub-cutaneous administration. It is also possible to administer the vaccine orally by mixing the active components with feed or water; providing a tablet form, etc.

Other means for administering the vaccine should be apparent to those skilled in the art from the teachings herein; accordingly, the scope of the invention is not limited to a particular delivery form.

It is also to be understood that the vaccine may include active components or adjuvants in addition to the antigen(s) or fragments thereof hereinabove described.

The invention will be further described with respect to the following examples; however, the scope of the invention is not to be limited thereby:

### EXAMPLES

### 1. Preparation of vaccine material:

Treponema hyodysenteriae strain B204 was grown in broth culture prepared as follows. Brain/Heart Infusion (Difco) at 37 g/liter distilled water was autoclaved allowed to cool, then sterile additions were made of a glucose solution (to a final concentration of 5 g/liter) and fetal calf serum (to final concentration of 5% vol/vol). The media was then prereduced (made anaerobic) by 24 hours of perfusion with a stream of gas composed of 90% nitrogen, 10% carbon dioxide. The complete media was then inoculated with a 1-10% volume of actively growing T. hyo culture, the temperature was maintained at 37°C-39°C, the culture pH was maintained at 6.8, and the culture was continuously perfused with the oxygen free gas (flow rate 50 ml/min/liter of culture).

Cells were removed from the fermentation when they had achieved a density of 10⁸/ml or greater (measured by microscopic count). Cells were concentrated by centrifugation then washed and recentrifuged twice in a buffer of 10mM potassium acetate pH 4.75, 150mM potassium chloride. The cells were then resuspended in 10mM potassium acetate pH 4.75 until an optical density of 25-30 (at 600mM) was achieved (as measured on solution dilutions) which is typically about 1/20 the original culture volume.

### Extraction method 1:

Tween-20® (a non-ionic detergent) was then added to the cell suspension to achieve a final concentration of 0.2%. After gentle agitation for 10 minutes the cells were centrifuged (10,000 xg for 10 min). This supernatant fraction was discarded and the cells were resuspended in acetate buffer and then extracted with 2.0% Tween-20®. After centrifugation the 2% Tween supernatant (detergent solubilized antigen pool) was saved and the cell pellet was resuspended and re-extracted with Tween-20 in a sequential manner for up to 5 additional cycles with the concentration increasing over the cycles from about 2% up to about 10%. The detergent solubilized supernatant fractions were pooled. This extraction procedure selectively (but not quantitatively) solubilizes surface proteins of T. hyo without lysing or rupturing the bacteria.

To reduce the detergent levels in the antigen preparation, 2 additional steps were used. First, the material was subjected to ultracentrifugation (100,000 x g) for 1.5 hours, and the recovered pellet material was resuspended in 25mM Tris buffer pH 6.8 and dispersed by sonication. The recovered supernatant fraction containing the bulk of detergent was passed over a column of DEAE - Sephacel® [Pharmacial] equilibrated in 10mM potassium acetate pH 4.75. The protein components bound to the chromatography resin while the non-ionic detergent was washed through. Proteins were released by elution in a high salt (1.0M NaCl) buffer, collected in 3ml fractions, appropriate fractions pooled and then dialyzed in 25mM Tris buffer pH 6.8.

These two pools of detergent depleted antigens were then pooled and brought to a protein concentration of 10 mg/ml. This material constitutes the array of proteins released from T. hyo cells (both soluble and insoluble forms) after extraction with Tween 20®.

The array of proteins contain T. hyo antigens having the following molecular weights: 29K; 30K; 31K; 34K; 36K; 38K; 39K; 42K; 44K; and 60K. In addition, the array of proteins includes some 19K antigen. Some of the antigens were sequenced and the sequence data is as follows:

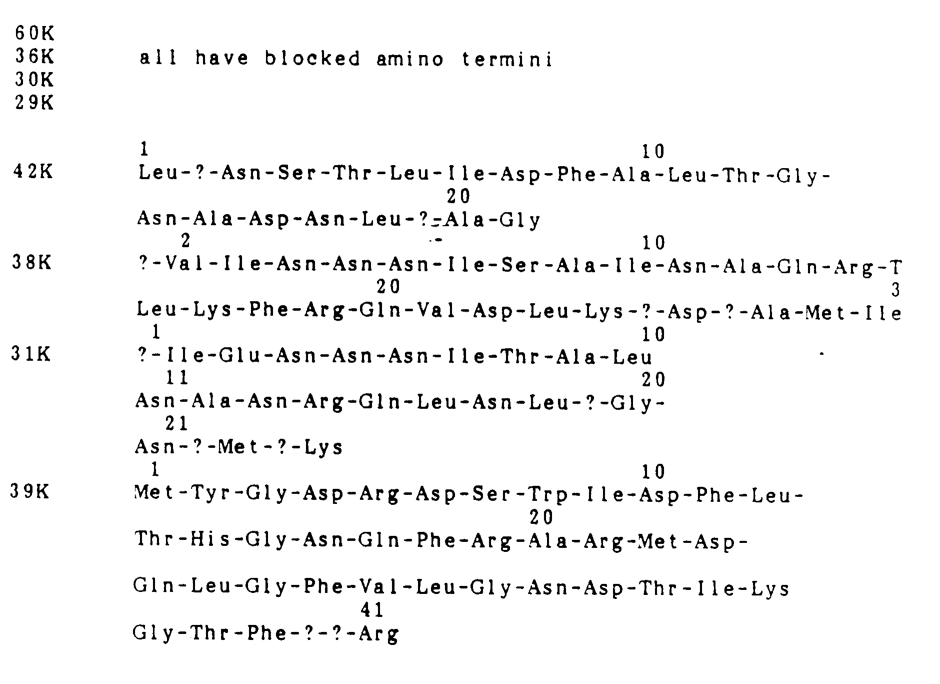

### Extraction method 2: Preparation of SDS Soluble Surface Proteins.

Cells are harvested, washed and resuspended as described above except that the resuspension buffer contains 0.9M sucrose and 20mM MgCl_{2.} A solution (20mM) of the ionic detergent sodium dodecyl sulfate, SDS, was added slowly with gentle agitation until the absorbance at 600mM of a 50-fold dilution of the cell suspension had decreased by approximately 30%. This typically occurs at a final SDS concentration of 15-20mM. Unlysed cells were separated from the detergent released proteins by low speed centrifugation at 10,000 x g for 10 min. This pool of protein (diluted to 10mg/ml) contains both soluble and insoluble components released by treating T. hyo cells with SDS.

### Antigen Purification

The recovered high speed pellet (HSP) obtained by ultracentrifugation for 1.5 hours in hereinabove extraction method 1 was resuspended in 25mM tris-HCl pH 6.8 and dispersed by sonication. The HSP was then mixed with 15 volumes of tris-HC1 pH 6.8 6M urea which had been filtered through a 0.45uM filter. This was stirred at room temperature for several hours. This was centrifuged at 100,000 xg and the supernatant (US1) set aside. The pellet fraction from this step (UP1) was resuspended and extracted with urea a second time. This material was centrifuged as before and the supernatant (US2) and pellet (UP2) were collected.

US1 was used to isolate the 29 kd, 34 kd, 42 kd and 44-45 kd proteins. This was done as follows:
US1 was acidified to 0.1% volume trifluoroacetic acid (TFA) and loaded directly onto a C4 reverse phase column equilibrated with 0.1% TFA. The column was developed with a gradient from 0-100% where 100% = acetonitrile + isopropanol (2:1) + 0.1% TFA. For preparative work using a 25mm x 250mm column a flow rate of 10ml/minute was used with the following gradient:
0-40% in 10'
40-52% in 65'
53-100% in 14'
at a collection rate of 0.9 min/fraction.

The eluant was monitored continuously at 214nm.

Under these conditions the following proteins were eluted:

| PEAK | ANTIGEN | % ACETONITRILE |
|---|---|---|
| 1 | 29 Kd | 43% |
| 2 | 29 Kd | 44% |
| 3 | 42 Kd | 45% |
| 4 | 34 Kd | 47% |
| 5 | 44 & 45 Kd | 49% |
| 6 | 45 | 50% |
| 7 | Multiple Proteins including 31 & 38 Kd | |

400 ml of US1 in 0.1% TFA (160mg of protein) can be loaded and give reasonable separation though there is some cross-contamination between Peaks 3 and 4.

Fractions containing the desired proteins were pooled, diluted with an equal volume of distilled water and lyophilized. Lyophilized fractions were resuspended in 25mM Tris-HCl, pH 6.8. This material was quantified, solubilized with TFA and loaded onto glass filters for derivatization and subsequent amino acid analysis on HPLC according to standard methods.

US2 was used to prepare the 29 and 38 KDa antigens as follows:
The material was acidified and loaded onto a C4 column as was the US1. A gradient was established as:
0-42% in 10ʹ
42-45% in 15ʹ
45-57% in 30ʹ
57-100% in 14ʹ
Under these conditions the following proteins were eluted:

| PEAK | ANTIGEN | % ACETONITRILE |
|---|---|---|
| 1 | 29 Kd | 45% |
| 2 | 38 Kd | 55% |

600ml of US2 (65mg of protein) has been loaded.

Fractions were pooled, diluted, lyophilized, resuspended and prepared for amino acid sequence analysis as above.

UP2 provided a source of 39 KDa protein which was purified by electro-elution from acrylamide gels.

Amino acid sequence determined for an HPLC purified peptide fragment derived from proteolytic digestion of the 39 kDa protein (of the UP2 cell fraction)using endoproteinase Lys-C. The 39 kDa protein was first precipitated with acetone and then resuspended in a solution of 4 M urea, 25 mM Tris pH. 6.8. One peptide was obtained as a peak of material eluting off of a C-4 reverse phase column developed with a gradient of acetonitrile, isopropanol (2:1) in 0.1% trifluoroacetic acid.

The 39 kDa fragment had the following sequence:

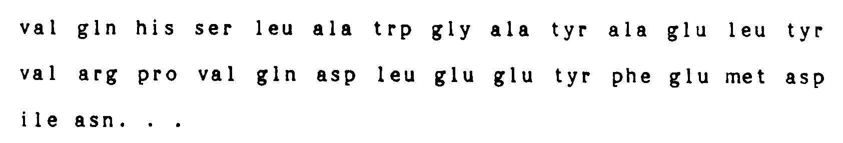

Amino acid sequence determined for the protease resistant component of the 39 kDa component of the UP2 fraction after its digestion with chymotrypsin (a suspension of 39 kDa protein at 2mg/ml was incubated at 37 degrees C for 16 hrs. with 20 »g/ml chymotrypsin in a buffer of 25 mM Tris, pH 6.8, containing 0.1% Zwittergent 3-12 detergent. A protease resistant 27 kDa product was isolated by electreolution after preparative gel electrophoresis and precipitated and extracted with methanol prior to sequencing. The compnent had the following sequence:

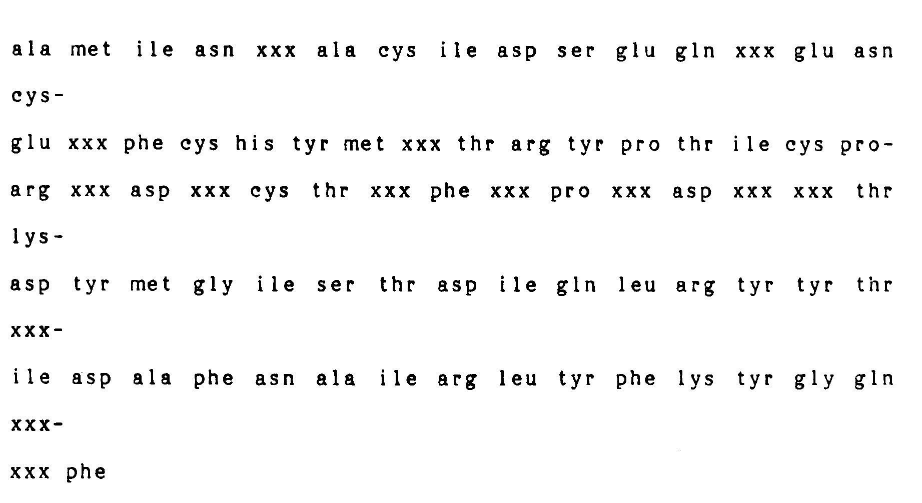

### EXAMPLE 2

Each vaccination dose was prepared by adding 2 mls of antigen (20mg of total protein) to 0.65ml of adjuvant (mix of purified mineral oil and paraffin), emulsifying by repeated passage through an 18g needle, and then intramuscular injection into an experimental pig. The antigens are obtained by either extraction method 1 or 2 of Example 1.

Eight week old pigs (approx. 40lbs (18,1 kg) each) were housed in isolated pens and fed a high protein, antibiotic free diet. Vaccinations were given every two weeks for a total of 3 injections per animal over a 6-week period.

### Oral challenge method:

Four weeks after the final vaccination, each animal received an oral challenge of 5.10¹⁰ virulent Treponema hyodysenteriae over a period of 4 days. Approximately half of the challenge was administered as broth culture passed through a stomach tube inserted in each animal. The other half of the challenge was presented as T. hyo cultures grown anaerobically in blood agar which was mixed with a small amount of feed and then completely consumed by each pig (all animals were fasted for 48 hours prior to oral challenge). Conditions used for broth culture of the T. hyo are virtually the same as described in section 1, except that Trypticose Soy broth (TSB) (27.5g/l) was substituted for the Brain/Heart infusion and the cultures were grown anaerobically with stirring but without perfusion of a continuous stream of gas.

Serum samples were collected throughout the experiment. Serum antibody in vaccinate animals directed against T. hyo proteins was demonstrated by Western blot and titer levels were shown to exceed those found in naturally immune (recovered) pigs as measured by ELISA assay.

Rectal swabs were cultured anaerobically to demonstrate the presence of T. hyo in the intestinal tract of challenged animals.

Daily examinations were made of each animal to evaluate general health, feces consistency, and to check for the presence of blood and mucous in fecal material.

Four animals were vaccinated as described above. Four unvaccinated, challenge control animals were housed in the same pen as the vaccinates and challenged at the same time using the same protocol.

Within 31 days after challenge all 8 animals showed clinically positive signs of infection with T. hyo. As summarized in Table I, three of the four vaccinates had a high serum titer prior to challenge and all three showed a significant delay (an additional 8-16 days) in onset of swine dysentery relative to the low titer vaccinate or the control group. The condition of vaccinates I, II and IV during clinical dysentery was better than that observed for the control animals during their period of dysentery. None of the vaccinates became moribund following challenge whereas two of the four control animals died from the infections.

Thus, the intramuscular vaccination of young pigs reduced the clinical severity of a subsequent swine dysentery infection and delayed the onset of such an outbreak.

**TABLE 1**

| | VACCINATES | | | | CONTROLS | | | |
|---|---|---|---|---|---|---|---|---|
| Animal # | I | II | III | IV | V | VI | VII | VII |
| Maximum anti-T. hyo Serum Titer | 500 | 500 | 500 | 200 | 20 | 20 | 20 | 20 |
| Antigen used | T | T | S | S | - | - | - | - |
| S.D. onset-days post challenge | 21 | 38 | 20 | 11 | 11 | 11 | 12 | 31 |
| Average condition during dysentery period (Table 2) | 1.9 | 1.25 | 2.8 | 1.6 | 2.1 | 2.4 | 3.0 | 1.0 |
| Death due to infection | No | No | No | No | No | Yes | Yes | No |
| T = Tween solubilized T. hyo proteins S = SDS solubilized T. hyo proteins | | | | | | | | |

**TABLE 2**

| Summary of pig scores | | | |
|---|---|---|---|
| | fecal consistency | fecal composition | pig condition |
| 1 | solid normal | normal | normal, alert |
| 2 | soft | slight amount of mucus | slightly gaunt slightly rough hair coat |
| 3 | very soft to loose | large amount of mucus flecks of blood | very gaunt very rough hair dull eyes |
| 4 | very loose watery | extremely bloody | moribund |

## Claims (Claims for the following Contracting State(s): AT, BE, CH, DE, FR, GB, IT, LI, LU, NL, SE)

1. A vaccine for protection against swine dysentery, comprising a Treponema hyodysenteriae antigen having a molecular weight of 19 to 90 kDa, or an active fragment thereof, the vaccine being essentially free of T. hyodysenteriae cells.

2. The vaccine of claim 1, wherein the antigen has a molecular weight of at least 25 kDa.

3. The vaccine of claim 2, wherein the antigen has a molecular weight of no greater than 65 kDa.

4. The vaccine of claim 1, wherein the antigen is selected from those of 29, 30, 31, 34, 36, 38, 39, 42, 44 or 60 kDa, and mixtures thereof.

5. The vaccine of claim 4, wherein the antigen is selected from those of 30, 36 or 60 kDa.

6. The vaccine of claim 4, wherein the antigen is selected from those of 29, 31, 34, 38, 39, 42 or 44 kDa.

7. The vaccine of any preceding claim, in the form of a unit dose containing at least 5 »g of the antigen.

8. The vaccine of claim 7, wherein the unit dose contains at least 100 »g of the antigen.

9. The vaccine of any preceding claim, wherein the antigen is as obtained by solubilising a T. hyodysenteriae organism with a detergent or chaotropic agent, without lysing or rupturing the organism.

10. Use of an antigen or active fragment thereof as defined in any preceding claim, for the manufacture of a medicament for protection against swine dysentery.

## Claims (Claims for the following Contracting State(s): ES, GR)

1. A method for preparing a vaccine for protection against swine dysentery, comprising formulating into a vaccine a Treponema hyodysenteriae antigen having a molecular weight of 19 to 90 kDa, or an active fragment thereof, the vaccine being essentially free of T. hyodysenteriae cells.

2. The method of claim 1, wherein the antigen has a molecular weight of at least 25 kDa.

3. The method of claim 2, wherein the antigen has a molecular weight of no greater than 65 kDa.

4. The method of claim 1, wherein the antigen is selected from those of 29, 30, 31, 34, 36, 38, 39, 42, 44 or 60 kDa, and mixtures thereof.

5. The method of claim 4, wherein the antigen is selected from those of 30, 36 or 60 kDa.

6. The method of claim 4, wherein the antigen is selected from those of 29, 31, 34, 38, 39, 42 or 44 kDa.

7. The method of any preceding claim, wherein the vaccine is in the form of a unit dose containing at least 5 »g of the antigen.

8. The method of claim 7, wherein the unit dose contains at least 100 »g of the antigen.

9. The method of any preceding claim, wherein the antigen is as obtained by solubilising a T. hyodysenteriae organism with a detergent or chaotropic agent, without lysing or rupturing the organism.

## Patentansprüche (Patentansprüche für folgende(n) Vertragsstaat(en): AT, BE, CH, DE, FR, GB, IT, LI, LU, NL, SE)

1. Impfstoff zum Schutz gegen Schweinedysenterie, umfassend ein Treponema hyodysenteriae-Antigen eines Molekulargewichts von 19 - 90 kDa oder ein aktives Fragment desselben, wobei der Impfstoff im wesentlichen von T. hyodysenteriae-Zellen frei ist.

2. Impfstoff nach Anspruch 1, wobei das Antigen ein Molekulargewicht von mindestens 25 kDa aufweist.

3. Impfstoff nach Anspruch 2, wobei das Antigen ein Molekulargewicht von nicht größer als 65 kDa aufweist.

4. Impfstoff nach Anspruch 1, wobei das Antigen aus solchen von 29, 30, 31, 34, 36, 38, 39, 42, 44 oder 60 kDa und Gemischen hiervon ausgewählt ist.

5. Impfstoff nach Anspruch 4, wobei das Antigen aus solchen von 30, 36 oder 60 kDa ausgewählt ist.

6. Impfstoff nach Anspruch 4, wobei das Antigen aus solchen von 29, 31, 34, 38, 39, 42 oder 44 kDa ausgewählt ist.

7. Impfstoff nach einem der vorhergehenden Ansprüche in Form einer Einheitsdosis mit mindestens 5 »g Antigen.

8. Impfstoff nach Anspruch 7, wobei die Einheitsdosis mindestens 100 »g des Antigens enthält.

9. Impfstoff nach einem der vorhergehenden Ansprüche, wobei das Antigen durch Löslichmachen eines T. hyodysenteriae-Organismus mit einem Detergens oder chaotropischen Mittel ohne Lyse oder Aufbrechen des Organismus erhalten wurde.

10. Verwendung eines Antigens oder aktiven Fragmens desselben gemäß der Definition nach einem der vorhergehenden Ansprüche zur Herstellung eines Medikaments zum Schutz gegen Schweinedysenterie.

## Patentansprüche (Patentansprüche für folgende(n) Vertragsstaat(en): ES, GR)

1. Verfahren zur Herstellung eines Impfstoffs zum Schutz gegen Schweinedysenterie, durch Überführen eines Treponema hyodysenteriae-Antigens eines Molekulargewichts von 19 - 90 kDa oder eines aktiven Fragments desselben in einen Impfstoff, der im wesentlichen von T. hyodysenteriae-Zellen frei ist.

2. Verfahren nach Anspruch 1, wobei das Antigen ein Molekulargewicht von mindestens 25 kDa aufweist.

3. Verfahren nach Anspruch 2, wobei das Antigen ein Molekulargewicht von nicht größer als 65 kDa aufweist.

4. Verfahren nach Anspruch 1, wobei das Antigen aus solchen von 29, 30, 31, 34, 36, 38, 39, 42, 44 oder 60 kDa und Gemischen hiervon ausgewählt ist.

5. Verfahren nach Anspruch 4, wobei das Antigen aus solchen von 30, 36 oder 60 kDa ausgewählt ist.

6. Verfahren nach Anspruch 4, wobei das Antigen aus solchen von 29, 31, 34, 38, 39, 42 oder 44 kDa ausgewählt ist.

7. Verfahren nach einem der vorhergehenden Ansprüche, wobei der Impfstoff in Form einer Einheitsdosis mit mindestens 5 »g Antigen vorliegt.

8. Verfahren nach Anspruch 7, wobei die Einheitsdosis mindestens 100 »g des Antigens enthält.

9. Verfahren nach einem der vorhergehenden Ansprüche, wobei das Antigen durch Inlösungbringen eines T. hyodysenteriae-Organismus mit einem Detergens oder chaotropischen Mittel ohne Lyse oder Aufbrechen des Organismus erhalten wurde.

## Revendications (Revendications pour l'(les) Etat(s) contractant(s) suivant(s): AT, BE, CH, DE, FR, GB, IT, LI, LU, NL, SE)

1. Vaccin destiné à conférer une protection contre la dysenterie porcine, comprenant un antigène de Treponema hyodysenteriae possédant un poids moléculaire de 19 à 90 kDa, ou un de ses fragments actifs, le vaccin étant pratiquement dépourvu de cellules de T. hyodysenteriae.

2. Vaccin suivant la revendication 1, dans lequel l'antigène possède un poids moléculaire d'au moins 25 kDa.

3. Vaccin suivant la revendication 2, dans lequel l'antigène possède un poids moléculaire non supérieur à 65 kDa.

4. Vaccin suivant la revendication 1, dans lequel l'antigène est choisi entre ceux de 29, 30, 31, 34, 36, 38, 39, 42, 44 et 60 kDa, ainsi que leurs mélanges.

5. Vaccin suivant la revendication 4, dans lequel l'antigène est choisi entre ceux de 30, 36 et 60 kDa.

6. Vaccin suivant la revendication 4, dans lequel l'antigène est choisi entre ceux de 29, 31, 34, 38, 39, 42 et 44 kDa.

7. Vaccin suivant l'une quelconque des revendications précédentes, sous forme d'une dose unitaire contenant au moins 5 »g de l'antigène.

8. Vaccin suivant la revendication 7, dans lequel la dose unitaire contient au moins 100 »g de l'antigène.

9. Vaccin suivant l'une quelconque des revendications précédentes, dans lequel l'antigène est tel qu'obtenu par solubilisation d'un micro-organisme T. hyodysenteriae avec un détergent ou agent chaotropique, sans lyse ou rupture du micro-organisme.

10. Utilisation d'un antigène ou d'un de ses fragments actifs telle que définie dans l'une quelconque des revendications précédentes, pour la production d'un médicament destiné à conférer une protection contre la dysenterie porcine.

## Revendications (Revendications pour l'(les) Etat(s) contractant(s) suivant(s): ES, GR)

1. Procédé de préparation d'un vaccin destiné à conférer une protection contre la dysenterie porcine, comprenant la formulation en un vaccin d'un antigène de Treponema hyodysenteriae possédant un poids moléculaire de 19 à 90 kDa, ou d'un de ses fragments actifs, le vaccin étant pratiquement dépourvu de cellules de T. hyodysenteriae.

2. Procédé suivant la revendication 1, dans lequel l'antigène possède un poids moléculaire d'au moins 25 kDa.

3. Procédé suivant la revendication 2, dans lequel l'antigène possède un poids moléculaire non supérieur à 65 kDa.

4. Procédé suivant la revendication 1, dans lequel l'antigène est choisi entre ceux de 29, 30, 31, 34, 36, 38, 39, 42, 44 et 60 kDa, ainsi que leurs mélanges.

5. Procédé suivant la revendication 4, dans lequel l'antigène est choisi entre ceux de 30, 36 et 60 kDa.

6. Procédé suivant la revendication 4, dans lequel l'antigène est choisi entre ceux de 29, 31, 34, 38, 39, 42 et 44 kDa.

7. Procédé suivant l'une quelconque des revendications précédentes, dans lequel le vaccin est sous forme d'une dose unitaire contenant au moins 5 »g de l'antigène.

8. Procédé suivant la revendication 7, dans lequel la dose unitaire contient au moins 100 »g de l'antigène.

9. Procédé suivant l'une quelconque des revendications précédentes, dans lequel l'antigène est tel qu'obtenu par solubilisation d'un micro-organisme T. hyodysenteriae avec un détergent ou agent chaotropique, sans lyse ou rupture du micro-organisme.
